# EUROPÄISCHE PATENTANMELDUNG

(11) **EP 1 975 600 A1**
(43) Veröffentlichungstag der Anmeldung: **01.10.2008**
(21) Anmeldenummer: 08006340.7
(22) Anmeldetag: 31.03.2008
(51) Int. Cl.: G01N 21/21, G01N 21/59, G01N 21/65, G01N 33/44

(54) **Verfahren zur Ermittlung des Vernetzungsgrades von Polymererzeugnissen**

(30) Priorität: 31.03.2007 DE 102007015667
(71) Anmelder: INOEX GmbH, 32547 Bad Oeynhausen (DE)
(72) Erfinder: Arnold, Christin, 33602 Bielefeld (DE); Deters, Martin, 49143 Bissendorf (DE)
(74) Vertreter: Seewald, Jürgen

(57) **Zusammenfassung**

Die vorliegende Erfindung betrifft ein Verfahren zur Ermittlung des Vernetzungsgrades von Polymererzeugnissen, insbesondere von extrudierten Rohren aus Polyethylen, wobei das Erzeugnis mit Licht bestrahlt wird, und der Vernetzungsgrad durch Analyse des von dem Polymererzeugnis beeinflussten Lichts ermittelt wird. Aufgabe der Erfindung ist es, ein weiteres derartiges Verfahren zur Ermittlung des Vernetzungsgrades von Polymererzeugnissen, insbesondere von extrudierten Rohren aus Polyethylen, zur Verfügung zu stellen. Gelöst wird diese Aufgabe dadurch, dass durch die Analyse die Kristallinität des Polymererzeugnisses als Maß für den Vernetzungsgrad erfasst wird.

## Beschreibung

Die vorliegende Erfindung betrifft ein Verfahren zur Ermittlung des Vernetzungsgrades von Polymererzeugnissen gemäß dem Oberbegriff des Anspruchs 1.

Polymererzeugnisse werden vernetzt, um ihnen gewünschte Eigenschaften zu verleihen. Insbesondere bei Polyethylenerzeugnissen kommen drei hauptsächliche Vernetzungsverfahren zur Anwendung, nämlich die Peroxid-, Silan- und die Strahlungsvernetzung. Bei allen drei Verfahren werden Polyethylenradikale erzeugt, die sich miteinander vernetzen bzw. zur Vernetzung beitragen.

Um festzustellen, ob bei der entsprechenden Behandlung der Polymererzeugnisse der erforderliche Vernetzungsgrad erreicht worden ist, müssen die Erzeugnisse einer Prüfung unterzogen werden. Dazu sind verschiedene Messverfahren bekannt, die sowohl an Proben im Rahmen einer Laborprüfung als auch während der Herstellung der Polymererzeugnisse durchgeführt werden.

In der DE 29 03 879 A1 wird ein Verfahren zur Messung des Vernetzungsgrades von Erzeugnissen aus vernetzten Polymerisaten beschrieben. Dieses Verfahren wird während der laufenden Produktion des Erzeugnisses durchgeführt, bei der sich das Erzeugnis bewegt. Bei diesem Verfahren wird das Erzeugnis senkrecht zu seiner Bewegungsrichtung mit einer Kraft beaufschlagt, wobei dann die Reaktionskomponente des Erzeugnisses herangezogen wird, um den Vernetzungsgrad des Erzeugnisses pro Querschnittsflächeneinheit zu ermitteln. Die Messung des Vernetzungsgrades erfolgt bei diesem Verfahren also auf mechanischem Wege.

Weitaus häufiger als mechanische kommen jedoch optische Verfahren zum Einsatz. So beschreibt die DE 197 07 967 A1 ein Verfahren zur Ermittlung des Vernetzungsgrades von Haftklebstoffschichten in Abhängigkeit von der Tiefe innerhalb der Haftklebstoffschicht. Bei diesem Verfahren erfolgt die Messung mittels konfokaler Ramanspektroskopie oder konfokaler Fluoreszenzspektroskopie. Dabei wird das an der zu untersuchenden Probe gestreute Ramanlicht bzw. Fluoreszenzlicht in Spektrallinien zerlegt und einem Fotodetektor zur Bestimmung der Intensität einer Spektrallinie zugeführt. Daraus kann dann der Vernetzungsgrad in der Haftklebstoffschicht ermittelt werden.

Ein weiteres optisches Verfahren ist in der JP 2006/214807 A beschrieben, welches ebenfalls im laufenden Produktionsprozess einsetzbar ist. Bei diesem Verfahren wird ein vernetztes Produkt mit einem Laserstrahl in einem Winkel von ungefähr 45° bestrahlt. Ein Teil der Laserstrahlung wird am Produkt reflektiert. Aus der Intensität des reflektierten Laserstrahls können dann Rückschlüsse auf den Vernetzungsgrad gezogen werden.

Aufgabe der vorliegenden Erfindung ist es, ein weiteres gattungsgemäßes Verfahren zur Ermittlung des Vernetzungsgrades von Polymererzeugnissen, insbesondere von extrudierten Rohren aus Polyethylen, zur Verfügung zu stellen.

Diese Aufgabe wird erfindungsgemäß mit einem Verfahren gelöst, welches die Merkmale des Anspruchs 1 aufweist.

Polymererzeugnisse haben sowohl kristalline als auch amorphe Strukturanteile. Die vorliegende Erfindung beruht nun auf der Erkenntnis, dass ein direkter Zusammenhang zwischen der Kristallinität und dem Vernetzungsgrad besteht, nämlich dahingehend, dass der Vernetzungsgrad umso größer ist, je geringer der kristalline Strukturanteil des Polymers ist und umgekehrt. Aus dem ermittelten Kristallinitätsgrad kann daher unmittelbar auf den Vernetzungsgrad geschlossen werden.

Weitere vorteilhafte Ausgestaltungen der Erfindung ergeben sich aus den Unteransprüchen.

Die Erfindung wird nachstehend anhand von Ausführungsbeispielen näher erläutert. In der dazugehörigen Zeichnung sind prinzipielle Meßaufbauten zur Durchführung des Verfahrens dargstellt. Im Einzelnen zeigt:
- Fig. 1: einen Aufbau zur Ermittlung der Kristallinität eines polymeren Erzeugnisses mittels der Ramanspektroskopie,
- Fig. 2: einen Aufbau zur Ermittlung der Kristallinität eines polymeren Erzeugnisses mittels des Durchlichtverfahrens, und
- Fig. 3: einen Aufbau zur Ermittlung der Kristallinität mittels des Verfahrens der Polarisationsmikroskopie.

In den Figuren der Zeichnung sind gleiche oder gleich wirkende Bauteile bzw. Merkmale mit gleichen Bezugszeichen versehen.

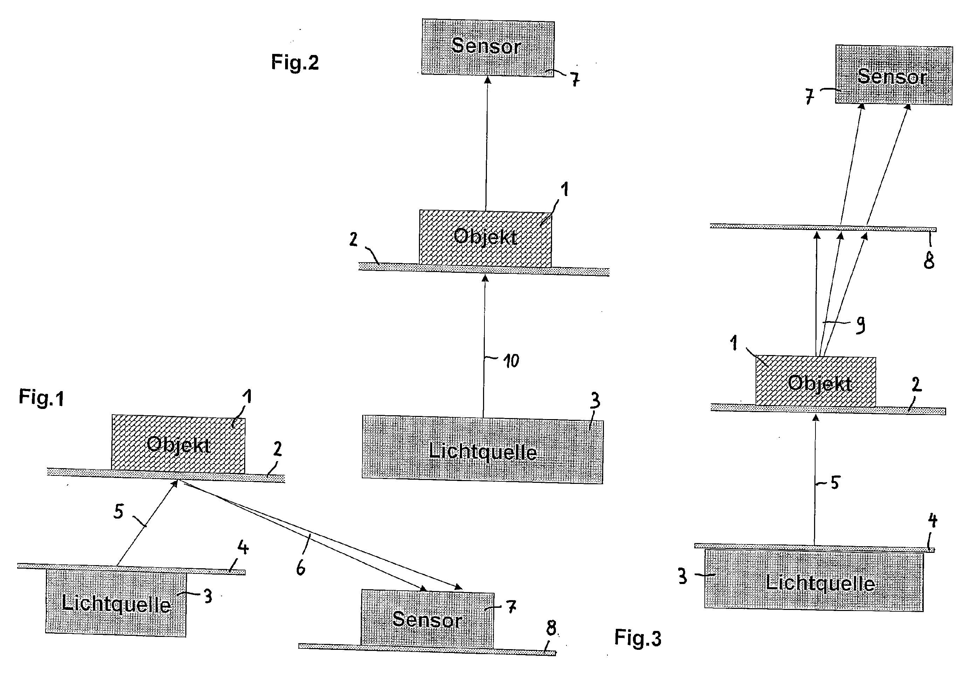

Mit dem Messaufbau gemäß Figur 1 wird der Kristallinitätsgrad eines aus einem polymeren Werkstoff bestehenden Objekts 1 mittels der Ramanspektroskopie ermittelt.

Das Objekt 1 liegt auf einem durchsichtigen Objekttisch 2, der beispielsweise aus Glas besteht. Dieser Objekttisch 2 kann natürlich zum Wegfall kommen, wenn das Objekt selbsttragend ist, z. B. ein extrudiertes Rohr, welches in einer Extrusionslinie an dem Meßaufbau vorbei läuft. Das gilt auch für die anderen Ausführungsbeispiele.

Das Objekt 1 wird mit linear polarisiertem Licht 5 bestrahlt. Um dies zu erreichen, ist vor einer Lichtquelle 3 ein Polarisationsfilter 4 angeordnet. Die Lichtquelle 3 strahlt einen Lichtstrahl 5 schräg auf das Objekt 1 ab, der an diesem gestreut wird. Das gestreute Licht 6 fällt in einen Sensor 7, in dem es in Abhängigkeit von der Wellenlänge spektral zerlegt wird. Das im Sensor 7 spektral zerlegte Licht durchläuft anschließend einen weiteren Polarisationsfilter 8, wodurch eine Bande für den kristallinen Anteil der Struktur des Kunststoffs und eine Bande für den amorphen Anteil der Struktur des Kunststoffs gebildet wird. Setzt man die von den Banden eingeschlossenen Flächen zueinander ins Verhältnis, so ergibt sich daraus der Kristallinitätsgrad, der ein Maß für den Vernetzungsgrad des Polymererzeugnisses ist.

Bei dem in Figur 2 gezeigten Messaufbau wird die Kristallinität eines Objekts 1 mittels eines Durchlichtverfahrens ermittelt. Dazu wird das Objekt 1 aus einer Lichtquelle 3 mit weißem Licht 10 bestrahlt, welches das Objekt 1 durchleuchtet. Voraussetzung dafür ist natürlich, dass das Objekt 1 lichtdurchlässig ist, was z. B. bei Polyethylenerzeugnissen der Fall ist, solange keine Farbstoffe eingemischt werden. Der das Objekt 1 verlassende Lichtstrahl 5 ist aufgrund der im Objekt 1 stattfindenden Absorption in seiner Intensität geschwächt. Die Intensität des Lichtstrahls 5 wird durch einen Sensor 7 gemessen. Je mehr der Lichtstrahl 4 durch das Objekt 1 abgeschwächt wird, umso geringer ist die Transparenz des Objekts 1. Je geringer die Transparenz des Objekts 1 ist, desto weniger kristalline Anteile sind in der Struktur des Objekts 1 enthalten. Aus der Transparenz des Objekts 1 kann daher daher unmittelbar auf den Kristallinitätsgrad und damit auf den Vernetzungsgrad des Objekts 1 geschlossen werden.

Figur 3 zeigt einen Messaufbau, bei dem die Kristallinität eines Objekts 1 mittels der Polarisationsmikroskopie ermittelt wird. Bei diesem Verfahren befindet sich zwischen dem Objekt 1 und der Lichtquelle 3 ein Polarisationsfilter 4, der bewirkt, dass das Objekt 1 nur von linear polarisiertem Licht 5 durchleuchtet wird. Durch das Objekt 1 ändert sich die Schwingungsebene des einfallenden Lichts 5. Es entsteht Licht 9 mehrerer Schwingungsebenen. Oberhalb des Objekts 1 befindet sich ein weiterer Polarisationsfilter 8, der nur das durch das Objekt 1 entstandene Licht durchlässt, nicht aber das linear polarisierte Licht 5, mit dem das Objekt 1 bestrahlt wird. Es entsteht dadurch ein farbiges Bild, das von einem Sensor 7 ausgewertet wird. Im Hinblick auf Kunststoff ist die Farbigkeit des entstehenden Bildes abhängig vom Kristallinitätsgrad des Kunststoffes. Zusätzlich ist aber auch die Anisotropie der Makromoleküle für die Farbigkeit des Bildes verantwortlich. Aus der Farbigkeit des Bildes kann also der Kristallinitätsgrad, und damit der Vernetzungsgrad, bestimmt werden, bei Ausschluss der Anisotropie der Makromoleküle.

Der in Figur 3 gezeigte Messaufbau kann auch für eine modifizierte Polarisationsmikroskopie verwendet werden. Bei diesem "Drehwinkel-Verfahren" genannten Verfahren wird der Drehwinkel des durch das Objekt 1 entstandenen Lichts 9 bestimmt, der ein Maß für die Dichte und somit auch für den Kristallinitätsgrad ist.

Bei dem oben beschriebenen Verfahren der Polarisationsmikroskopie sieht der Sensor 7, z. B. eine Kamera oder eine Person, bei Beleuchtung des Objekts 1 ein farbiges Bild. Wird nun der Polarisationsfilter 8 um einen bestimmten Verdrehwinkel verdreht, so sieht der Sensor 7 nur noch eine schwarze Fläche, da durch die neue Position des Polarisationsfilters 8 alle Lichtstrahlen ausgelöscht worden sind. Der Verdrehwinkel des Polarisationsfilters 8 ist messbar. Er ist proportional zum Drehwinkel des durch das Objekt 1 entstandenen Lichts.

Eine weitere Möglichkeit zur Bestimmung des Kristallinitätsgrades eines polymeren Erzeugnisses ist mit einem "Steigung-Verfahren" genanntes Verfahren realisierbar. Bei diesem Verfahren wird ein Objekt mit unterschiedlicher Wellenlänge bestrahlt, wobei wohl die Transmission als auch die Reflektion zur Anwendung kommen kann. Es kann dabei beispielsweise Licht angewendet werden, beginnend bei einer Wellenlänge von 400 nm bis zu einer Wellenlänge von 900 nm. Unabhängig davon, ob nun Transmission oder Reflektion des Lichts angewendet worden ist, nimmt die Intensität des Lichts wegen der Absorption im Kunststoff ab. Allerdings nimmt die Intensität bei unterschiedlichen Wellenlängen in unterschiedlichen Maßen ab. Man erhält einen Graph, der die Intensität des transmittierten/reflektierten Strahls in Abhängigkeit von dessen Wellenlänge darstellt. Die Steigung dieses Graphen ist ein Maß für den Kristallinitätsgrad des Objekts.

## Patentansprüche

1. Verfahren zur Ermittlung des Vernetzungsgrades von Polymererzeugnissen, insbesondere von extrudierten Rohren aus Polyethylen, wobei das Erzeugnis mit Licht bestrahlt wird, und der Vernetzungsgrad durch Analyse des von dem Polymererzeugnis beeinflussten Lichts ermittelt wird, **dadurch gekennzeichnet, dass** durch die Analyse die Kristallinität des Polymererzeugnisses als Maß für den Vernetzungsgrad erfasst wird.

2. Verfahren nach Anspruch 1, **dadurch gekennzeichnet, dass** die Kristallinität mittels der Ramanspektroskopie ermittelt wird.

3. Verfahren nach Anspruch 2, **dadurch gekennzeichnet, dass**
- das Erzeugnis (1) mit linear polarisiertem Licht (5) bestrahlt wird
- das am Erzeugnis (1) gestreute Licht (6) in Abhängigkeit von der Wellenlänge in einem Sensor (7) spektral zerlegt wird
- das spektral zerlegte Licht anschließend einen Polarisationsfilter (8) zur Abbildung einer Bande für den kristallinen Anteil in der Struktur des Erzeugnisses (1) und eine Bande für den amorphen Anteil in der Struktur des Erzeugnisses (1) durchläuft, wobei sich der Kristallinitätsgrad aus den Flächenverhältnissen der beiden Banden ergibt.

4. Verfahren nach Anspruch 1, **dadurch gekennzeichnet, dass** der Kristallinitätsgrad mittels eines Durchlichtverfahrens ermittelt wird.

5. Verfahren nach Anspruch 4, **dadurch gekennzeichnet, dass**
- das Erzeugnis (1) mit weißem Licht (11) durchleuchtet wird
- die Transparenz des Erzeugnisses (1) anhand des aus dem Erzeugnis (1) austretenden Lichts (5) durch einen Sensor (7) gemessen wird
- aus der Transparenz auf den Kristallinitätsgrad geschlossen wird.

6. Verfahren nach Anspruch 1, **dadurch gekennzeichnet, dass**
- Das Erzeugnis (1) mit Licht unterschiedlicher Wellenlänge bestrahlt wird
- die Intensität des reflektierten/transmittierten Strahls in Abhängigkeit von der Wellenlänge von einem Sensor erfasst wird
- aus den Messdaten ein Graph gebildet wird, der die Intensität des reflektierten/transmittieren Strahls in Abhängigkeit von der Wellenlänge darstellt
- aus der Steigung des Graphen auf den Kristallinitätsgrad des Erzeugnisses geschlossen wird.

7. Verfahren nach Anspruch 1, **dadurch gekennzeichnet, dass** der Kristallinitätsgrad mittels der Polarisationsmikroskopie ermittelt wird.

8. Verfahren nach Anspruch 7, **dadurch gekennzeichnet, dass** aus der Farbigkeit des entstehenden Bildes des Erzeugnisses (1) auf dessen Kristallinitätsgrad geschlossen wird.

9. Verfahren nach Anspruch 1, **dadurch gekennzeichnet, dass** der Kristallinitätsgrad eines Erzeugnisses mit einer modifizierten Polarisationsmikroskopie ermittelt wird, indem die durch das Erzeugnis verursachte Drehung der Schwingungsebene des Lichts bestimmt wird, die ein Maß für die Dichte und somit für den Kristallinitätsgrad ist.
